# EUROPEAN PATENT APPLICATION

(11) **EP 3 000 384 A1**
(43) Date of publication of application: **30.03.2016**
(21) Application number: 15184116.0
(22) Date of filing: 07.09.2015
(51) Int. Cl.: A61B 3/12, A61B 3/10, A61B 3/14, G03B 15/05

(54) **OPHTHALMOSCOPE SYSTEM FOR ENHANCED IMAGE CAPTURE**

(30) Priority: 08.09.2014 US 201414479650
(71) Applicant: Eyenez, LLC, Glendora, CA 91741 (US)
(72) Inventor: Kislinger, Mark B., SAN MARINO, CA California 91108 (US); Varga, Emerick S., PASADENA, CA California 91103 (US)
(74) Representative: Ström & Gulliksson AB

(57) **Abstract**

A system (100) comprising an ophthalmoscope (102) having a proximal end (108), a distal end (110), and a body portion (112) with a light receptacle. The system includes a control assembly (104) having a first side (204) with a coupler (210) configured to engage the proximal end of the ophthalmoscope and an extension arm configured to engage the body portion (112) of the ophthalmoscope. The control assembly further includes a second side (214) configured to engage a computing device (106) having a camera and a camera flash. The control assembly further includes a sensor (220) configured to detect light from the camera flash, a light source (218) configured to be received in at least a portion of the light receptacle, and a circuit board (226) configured to operatively connect the sensor (220) and the light source (218). The circuit board (226) is configured to trigger the light source (218) in response to the camera flash.

## Description

### BACKGROUND

Traditional diagnostic instruments for examining an eye may include an ophthalmoscope, also referred to as a funduscope. To evaluate the health of the eye and identify eye disorders, the ophthalmoscope allows a user such as medical professional to see internal structures of the eye. Typical ophthalmoscopes include a light source to allow visualization of these internal structures. However, traditional light sources as part of the ophthalmoscope may be insufficient to capture a diagnostic image of the eye using a camera of a computing device without using medication to dilate the eye. Traditional ophthalmoscopes further lack the ability to selectively adjust a light intensity of the light source. Moreover, typical ophthalmoscopes lack the ability to control a lighting sequence to capture a diagnostic image of the eye in a fully dilated state without medication. Additionally, traditional ophthalmoscopes may not include telemedicine capabilities to allow the transfer of diagnostic images between a plurality of computing devices.

It may be desirable to provide an improved system that is configured to minimize constriction of the eye to capture of a diagnostic image of an eye in a dilated condition (e.g., fully dilated) without using medication to dilate the eye. The improved system may provide an enhanced light source, a selectively adjustable light intensity, a controllable lighting sequence, and telemedicine capabilities for the transfer of diagnostic images between two or more computing devices. This improved system may further provide improved diagnostics for enhanced detection of eye disorders as well as early detection of other bodily disorders that have complications resulting in eye disorders.

### BRIEF DESCRIPTION OF THE DRAWINGS

Figure 1 illustrates a perspective view of an exemplary system of the present disclosure;
Figure 2 illustrates a side view of the exemplary system of Figure 1;
Figure 3 illustrates a front perspective view of an exemplary control assembly; and
Figure 4 illustrates a rear perspective view of the exemplary control assembly of Figure 3.

### DETAILED DESCRIPTION

An exemplary system may comprise an ophthalmoscope having a proximal end, a distal end, and a body portion with a light receptacle, the ophthalmoscope being configured to provide a view of an eye. The system may include a control assembly having a first side having a coupler configured to engage the proximal end of the ophthalmoscope and an extension arm configured to engage the body portion of the ophthalmoscope. The control assembly may further include a second side configured to engage a computing device having a camera with a camera flash being configured to capture an image (e.g., a diagnostic image) of the eye through the view of the ophthalmoscope. The control assembly may additionally include a sensor configured to detect light of the camera flash, a light source configured to be received in at least a portion of the light receptacle, and a circuit board configured to operatively connect the sensor and the light source. The circuit board may be configured to trigger the light source in response to the camera flash.

Methods of using the system are also contemplated. An exemplary method may comprise providing a view of an eye with an ophthalmoscope, detecting light from a camera flash of a computing device, triggering a light source associated with the ophthalmoscope in response to the camera flash, capturing an image of the view of the eye in conjunction with the camera flash with a camera of the computing device, storing the image as part of the computing device, and transmitting the image to another computing device with a transceiver of the computing device.

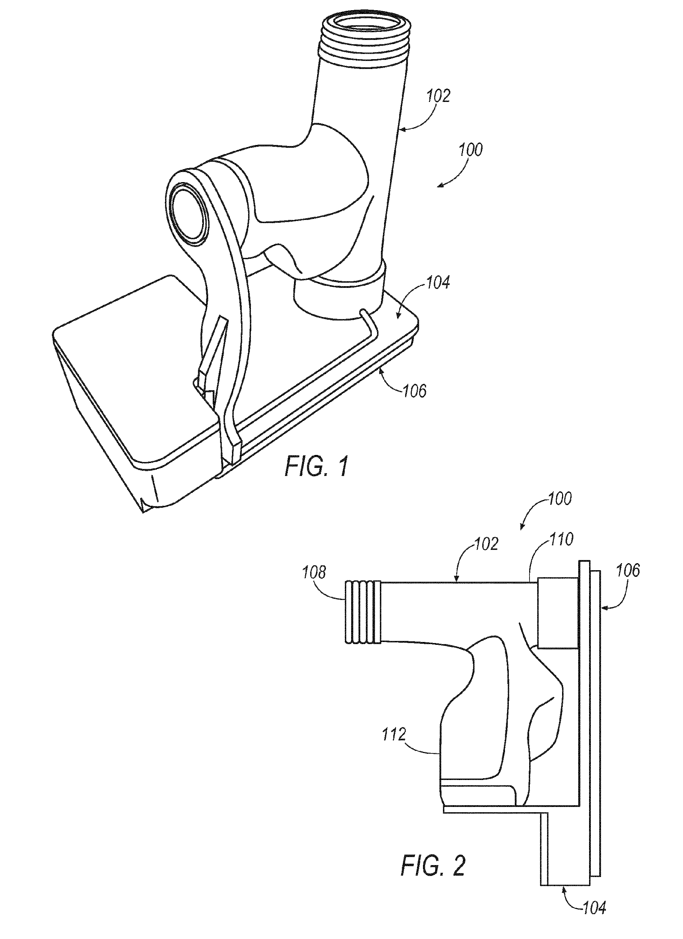

Figures 1-2 illustrate an exemplary system 100, for example, configured to identify eye disorders. System 100 may take many different forms and include multiple and/or alternate components and facilities. While an exemplary system 100 is shown in Figure 1, the exemplary components illustrated in Figure 1 are not intended to be limiting. Indeed, additional or alternative components and/or implementations may be used.

As shown in Figure 1, an exemplary system 100 may include a diagnostic instrument 102, a control assembly 104, and a computing device 106. The system 100 may be configured to facilitate examination of an eye, for example, to identify, image or photograph, and diagnose an eye disorder by viewing inner structures of the eye including, for example, the retina, optic disc, macula, and posterior pole, also called the fundus. By providing a view of the inner structures of the eye, the system 100 may be utilized to diagnose eye disorders.

The system 100 may be configured to identify any eye disorder, for example, during an eye examination by a user such as an ophthalmologist, optometrist, optician, eye technician, or any other medical professional. An eye disorder may include, for example, any disorder that is viewable from the inner structures of the eye. Exemplary eye disorders may include any disorder affecting the normal function of the eye, for example, an eye disease, damage to the eye (e.g., resulting from trauma or another bodily disease), or any other vision disorder. Exemplary eye disorders may include, without limitation, diabetic retinopathy, age-related macular degeneration (AMD), allergies, amblyopia (also referred to as "lazy eye"), astigmatism, bacterial keratitis, cataracts, conjunctivitis (also referred to as "pink eye"), detached and torn retina, dry eye, floaters and flashes, glaucoma, low vision, and presbyopia (also referred to as "aging eye"). Accordingly, the system 100 may be utilized to identify and diagnose any condition affecting normal function of the eye.

Furthermore, the system 100 may be configured to identify other bodily disorders, for example, during a physical examination by a user such as general medical practitioner or any other medical professional. The system may be configured to detect complications viewable from the inner structures of the eye. For example, the system 100 may be utilized to identify diabetic retinopathy of the eye resulting from diabetes. The system 100 may be utilized to identify hypertension, glaucoma, papilledema, and any other bodily disorder affecting the eye.

The diagnostic instrument 102 may include any device configured to view the eye. An exemplary diagnostic instrument 102 may include an ophthalmoscope. As shown in Figure 2, the diagnostic instrument 102 may include a proximal end 108 configured to be positioned near the user during examination and through which the user examines the eye. The diagnostic instrument 102 may further include a distal end 110 configured to be engaged by the control assembly 104 and positioned toward the eye during examination. The diagnostic instrument 102 may also include a body portion 112 such as a handle, which may be held by the user. The body portion 112 may include a light receptacle configured to receive a light source. The diagnostic instrument 102 may additionally include a mirror that reflects light from the light source into the eye, a viewing aperture through which the eye is examined, and a dial holding several lenses of varying strengths, which are used to allow the user to manually focus and visualize of structures of the eye at multiple depths. The light source may be included as part of the diagnostic instrument or as part of the control assembly 104.

The control assembly 104, as described in further detail below, may be configured to operatively connect the diagnostic instrument 102 and the computing device 106. Any or all of the diagnostic instrument 102, control assembly 104, and computing device 106 may be modular, selectively or permanently attachable, or integrated with respect to each other. The control assembly 104 may be configured to physically align the camera of the computing device 106 with the viewing aperture of the diagnostic instrument 102. The control assembly 104 may include the light source of the diagnostic instrument 102. The control assembly 104 may be configured to adjust a light intensity of the light source, trigger the light source in response to the camera flash of the computing device 106, and control the lighting sequence to image the eye in a dilated condition (e.g., fully dilated) without using medication to dilate the eye. Thus, the control assembly 104 may be configured to operably integrate the diagnostic instrument 102 and the computing device 106.

The computing device 106 may include a processor, a memory, a display, a transceiver, a camera, and a camera flash. The computing device 106 may include, without limitation, any mobile device, cellular phone, smart-phone, super-phone, tablet computer, handheld computer, notebook, laptop, electronic camera, or any other computing system and/or device. For example, a cellular phone may include any device configured to communicatively connect with a telecommunication network. The computing device 106 may include autofocus to automatically focus or manual focus to manually focus the camera relative to the internal structures of the eye (e.g., using the processor), capture an image of the eye (e.g., using the camera), store the image of the eye (e.g., as part of the memory), and transfer the image, e.g., using a wireless or wired connection, to one or a plurality of other computing devices (e.g., using the transceiver or a data cable). The autofocus and manual focus of the computing device 106 may be in conjunction with the manual focus of the diagnostic instrument 102. The computing device 106 may further be configured to provide light, e.g., using the camera flash, in conjunction with the capture of the image. The computing device 106 may be configured to present, e.g., as part of the display, the image captured by the camera. The display (e.g., a touchscreen) or a depressible button of the computing device 106 may initiate capture of the image.

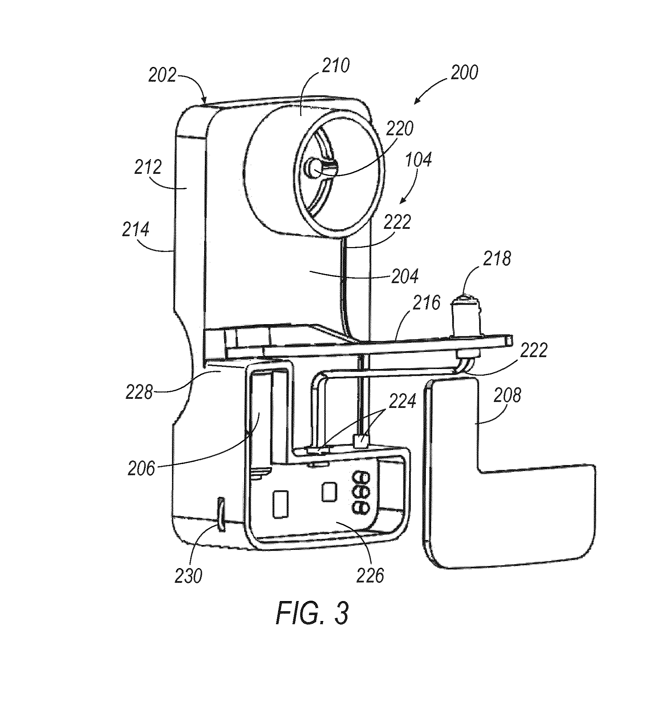

Figures 3-4 illustrate an exemplary system 200 including, for example, a control assembly 104. System 200 may take many different forms and include multiple and/or alternate components and facilities. While an exemplary system 200 is shown in Figures 3-4, the exemplary components illustrated in Figures 3-4 are not intended to be limiting. Indeed, additional or alternative components and/or implementations may be used.

As illustrated in Figures 3-4, system 200 may include a housing 202, a first side 204, a protrusion 206, a cover 208, a coupler 210, a side surface 212, a second side 214, and an extension arm 216. As shown in Figure 2a, system 200 may further include a light source 218, a sensor 220, connections 222, connectors 224, a circuit board 226, a power source 228, and a light control 230. As further shown in Figure 2b, system 200 may include a sensor aperture 231, a camera aperture 232, retainer walls 234a-c, and a recess 236.

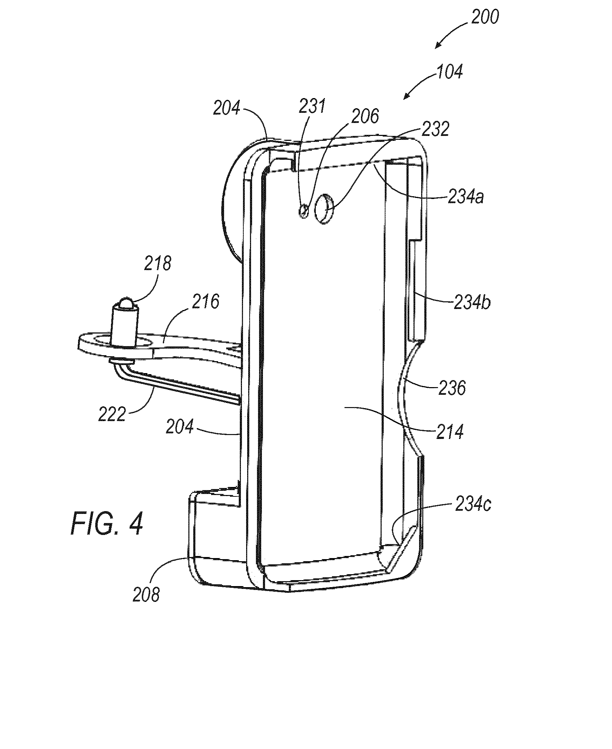

The system 200 may be configured as a physical interface between the diagnostic instrument 102 and the computing device 106. The system 200 may include the housing 202 having the first side 204 (e.g., Figure 3) facing the diagnostic instrument 102, the side surface 212 connecting the first side 204 and the second side 214, and the second side 214 (e.g., Figure 4) facing the computing device 106. The system 200 may include the protrusion 206 and the cover 208, for example, to contain the circuit board 226 and power source 228. The system 200 may further include the coupler 210 to align the viewing aperture of the diagnostic instrument 102 with the camera of the computing device 106. The housing 202 may also include an extension arm 216 to align the light source 218 with the diagnostic instrument 102. The housing 202 may also include retainer walls 234a-c to engage the computing device 106 and recess 236 to facilitate removal of computing device 106 from the housing 202.

The system 200 may be configured to selectively activate the light source 218. The light source 218 manually or automatically activated by the circuit board 226. For example, the light control 230 may manually adjust an amount of power applied to the light source 218. Furthermore, the sensor 220 may automatically activate the light source 218 in response to a camera flash of the computing device 106, which may be at full power or the amount of power set by the light control 230.

The light source 218 may include any device configured to emit light, for example, in conjunction with the camera of the computing device 106. The light source 218 may be configured to emit light of any wavelength. An exemplary light source 218 may include a light emitting diode (LED), an infrared (IR) light source, a halogen light source, an incandescent light source, or any combination thereof. The light source 218 may be configured to emit light at a light intensity or brightness based on the circuit board 226. The light source 218 may be powered by the circuit board 226 with a selected light intensity based on the light control 230, activated by the circuit board 226 at a full light intensity or the selected light intensity in response to light from a camera flash of the computing device 106 detected by the sensor 220, or a combination thereof. The light source 128 may be configured to provide constant light (e.g., for a predefined or a user-determined time period and light intensity), intermittent light (e.g., at a predefined frequency and light intensity), a flash (e.g., having a predetermined duration and light intensity), or a combination thereof.

The sensor 220 may include any sensor of light or electromagnetic energy. An exemplary sensor 220 may include a photocell, photo resistor, photodiode, reverse-biased LED, or charge-coupled device. The sensor 220 may be configured to detect light from the camera flash of the computing device 106. The sensor 220 may communicate, using connection 222, presence of light from the camera flash to the circuit board 226, using connector 224.

The circuit board 226 may be configured to operatively connect the sensor 220 with the light source 218. The circuit board 226 may be configured to detect the camera flash using the sensor 220 and, in response, activate the light source 218, e.g., using connection 222. The circuit board 226 may activate the light source 218 based on the amount of power set by the light control 230, in response to light from the camera flash being received by the sensor 220, or a combination thereof.

The power source 228 may include any device configured to provide power to the light source 218 and circuit board 226. An exemplary power source 228 may include a DC power source, an AC power source, or a combination thereof. An exemplary DC power source may include a battery, for example a lithium-ion, nickel-cadmium, alkaline, lithium, nickel oxyhydroxide, or silver-oxide battery. The power source 228 may be rechargeable or non-rechargeable.

The light control 230 may include any device configured to adjust an amount of power provided to the light source 218 from the power source 228. An exemplary light control 230 may include an adjustable voltage divider, for example an analog or digital potentiometer with a sliding or rotating contact. The light control 230 may control a light intensity or brightness of the light source 218, for example, to adjust the amount of light used in conjunction with the image captured by the camera of the computing device 106. The light control 230 may be selectively adjusted to provide a lower or an intermediate light intensity, for example, to promote full dilation of the eye. The light control 230 may be selectively adjusted to provide a full light intensity, for example, to promote capture of an image by the camera of the computing device 106.

In use, the system 200 may be configured to capture an image of the eye, for example, in a dilated (e.g., fully dilated) condition without using medication to dilate the eye. The system 200 may utilize a lighting sequence to optimize dilation of the eye without medication. For example, the light source 218 may be powered a lower or intermediate light intensity to position the system 200 relative to the eye or to examine the eye while promoting a dilated condition of the eye without medication. With full dilation of the eye, the camera of the computing device 106 may capture an image of the eye with the light source 218 at a higher or full intensity. As a further example, the circuit board 226 may be configured to delay a flash of the light source 218 to limit the time period that the eye has to constrict in response to the camera flash of the computing device 106. In an additional example, the light source 218 may include an infrared light source that may not be perceptible to the eye to promote full dilation of the eye and, in response to a full dilation of the eye, the camera of the computing device 106 may utilize autofocus or manual focus, e.g., without a flash, to capture the image of the eye.

With regard to the processes, systems, methods, heuristics, etc. described herein, it should be understood that, although the steps of such processes, etc. have been described as occurring according to a certain ordered sequence, such processes could be practiced with the described steps performed in an order other than the order described herein. It further should be understood that certain steps could be performed simultaneously, that other steps could be added, or that certain steps described herein could be omitted. In other words, the descriptions of processes herein are provided for the purpose of illustrating certain embodiments, and should in no way be construed so as to limit the claims.

Accordingly, it is to be understood that the above description is intended to be illustrative and not restrictive. Many embodiments and applications other than the examples provided would be apparent upon reading the above description. The scope should be determined, not with reference to the above description, but should instead be determined with reference to the appended claims, along with the full scope of equivalents to which such claims are entitled. It is anticipated and intended that future developments will occur in the technologies discussed herein, and that the disclosed systems and methods will be incorporated into such future embodiments. In sum, it should be understood that the application is capable of modification and variation.

All terms used in the claims are intended to be given their broadest reasonable constructions and their ordinary meanings as understood by those knowledgeable in the technologies described herein unless an explicit indication to the contrary in made herein. In particular, use of the singular articles such as "a," "the," "said," etc. should be read to recite one or more of the indicated elements unless a claim recites an explicit limitation to the contrary.

The Abstract of the Disclosure is provided to allow the reader to quickly ascertain the nature of the technical disclosure. It is submitted with the understanding that it will not be used to interpret or limit the scope or meaning of the claims. In addition, in the foregoing Detailed Description, it can be seen that various features are grouped together in various embodiments for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separately claimed subject matter.

## Claims

1. A system comprising:
an ophthalmoscope having a proximal end, a distal end, and a body portion with a light receptacle, the ophthalmoscope being configured to provide a view of an eye; and
a control assembly having a first side having a coupler configured to engage the proximal end of the ophthalmoscope and an extension arm configured to engage the body portion of the ophthalmoscope and having a second side configured to engage a computing device having a camera and a camera flash, the camera being configured to capture an image of the eye through the view of the ophthalmoscope, the control assembly further including:
a sensor configured to detect light of the camera flash;
a light source configured to be received in at least a portion of the light receptacle; and
a circuit board configured to operatively connect the sensor and the light source, wherein the circuit board is configured to trigger the light source in response to the camera flash.

2. The system of claim 1, wherein the sensor includes a photocell.

3. The system of claim 1 or 2, wherein the light source is triggered at a full intensity.

4. The system of any of the preceding claims, wherein the control assembly further includes a light control configured to control a light intensity of the light source.

5. The system of claim 4, wherein the light source is triggered at an intermediate intensity determined by the light control.

6. The system of any of the preceding claims, wherein the light source includes at least one of an LED and an infrared light source.

7. The system of any of the preceding claims, wherein the computing device is configured to send the image to a plurality of other computing devices.

8. A control assembly comprising:
a first side having a coupler configured to engage a proximal end of the ophthalmoscope and an extension arm with a light source configured to be received in a body portion of the ophthalmoscope;
a second side opposing the first side and being configured to engage a computing device having a camera flash;
a sensor adjacent the second side and being configured to detect light from the camera flash; and
a circuit board adjacent the first side and configured to trigger the light source in response to the sensor.

9. The assembly of claim 8, wherein the sensor includes a photocell.

10. The assembly of claim 8 or 9, wherein the light source is triggered at a full intensity.

11. The assembly of any of claims 8 to 10, further comprising a light control configured to control a light intensity of the light source.

12. The assembly of claim 11, wherein the light source is triggered at an intermediate intensity determined by the light control.

13. The assembly of any of claims 8 to 12, wherein the light source includes at least one of an LED and an infrared light source.

14. The assembly of any of claims 8 to 13, further comprising a battery operatively connected to the light source.

15. A method comprising:
providing a view of an eye with an ophthalmoscope;
detecting, by way of a sensor, light from a camera flash of a computing device;
triggering, by way of a circuit board, a light source associated with the ophthalmoscope in response to the camera flash;
capturing an image of the view of the eye with a camera of the computing device;
storing the image as part of the computing device; and
transmitting the image to another computing device.

16. The method of claim 15, wherein the sensor includes a photocell.

17. The method of claim 15 or 16, wherein the light source includes at least one of an LCD and an infrared light source.

18. The method of any of claims 15 to 17, wherein the light source is triggered at a full intensity.

19. The method of any of claims 15 to 18, further comprising:
controlling, by way of a light control, a light intensity of the light source.

20. The method of claim any of claims 15 to 19, wherein the light source is powered by a battery connected to the circuit board.
